# EUROPEAN PATENT APPLICATION

(11) **EP 4 454 670 A1**
(43) Date of publication of application: **30.10.2024**
(21) Application number: 23174684.3
(22) Date of filing: 22.05.2023
(51) Int. Cl.: A61L 27/46, A61L 27/54, C07K 17/00

(54) **POLYMER-CERAMIC BIOCOMPOSITES WITH PRO-REGENERATIVE PROPERTIES FOR FILING BONE DEFECTS AND REGENERATING BONE TISSUE AND METHOD OF OBTAINING THEM**

(30) Priority: 28.04.2023 PL 44287723
(71) Applicant: Siec Badawcza Lukasiewicz - Instytut Ceramiki i Materialow Budowlanych, Krakow (PL); Uniwersytet Gdanski, 80-309 Gdansk (PL); Politechnika Wroclawska, 50-370 Wrocklaw (PL); Instytut Biotechnologii I Medycyny Molekularnej, 80-180 Gdansk (PL); SENSDX SA, 02-676 Warszawa (PL); Uniwersytet Lodzki, 90-136 Lodz (PL)
(72) Inventor: Gzainska, Malgorzata, Jeszkowice (PL); Krokos, Anna, Wroclaw (PL); Otryl, Ewelina, Lisowice (PL); Grzymajlo, Michal, Wroclaw (PL); Szustakiewicz, Konrad, Wroclaw (PL); Kobielarz, Magdalena, Domaszczyn (PL); Chyzy, Katarzyna, Wroclaw (PL); Kubis, Agnieszka, Gdansk (PL); Karska, Natalia, Gdynia (PL); Sawicka, Justyna, Gdansk (PL); Rodziewicz-Motowidlo, Sylwia, 83-010 Rotmanka (PL); Ciolek, Lidia, Warszawa (PL); Biernat, Monika, Warszawa (PL); Szternet, Piotr, Warszawa (PL); Wozniak, Anna, Warszawa (PL); Jaegermann, Zbigniew, Spychowo (PL); Rudnicka, Karolina, Lodz (PL); Plocinski, Przemyslaw, Lodz (PL); Szwed-Georgiou, Aleksandra, Lodz (PL); Wlodarczyk, Marcin, Lodz (PL); Krupa, Agnieszka, Lodz (PL)
(74) Representative: Pawlowska-Bajerska, Justyna

(57) **Abstract**

The invention are ternary polymer-ceramic bio-composites with pro-regenerative properties produced in the form of flexible solid materials based on poly(glycerol adipate) containing an engineered peptide with a specific amino acid sequence hereinafter referred to as "UG51", with proven pro-regenerative properties, and as a ceramic phase one selected type of bioglass particles chosen from two: non-surface-modified bioglass, L-lysine surface-modified bioglass. These are bioactive composites with pro-regenerative properties and are applicable for filling bone defects and for regenerating bone tissue. Also, an object of the invention is a method of preparation bioactive polymer-ceramic composites with proven pro-regenerative properties for use in filling bone defects and regenerating bone tissue.

## Description

The invention refers to three-component polymer-ceramic bio-composites with pro-regenerative properties produced as flexible solid materials based on poly(glycerol adipate) containing an engineered peptide with a specific amino acid sequence hereinafter referred to as "UG51", with proven pro-regenerative properties, and as a ceramic phase one selected type of bioglass particles chosen from two: non-surface-modified bioglass, L-lysine surface-modified bioglass. These are bioactive composites with pro-regenerative properties and are applicable for filling bone defects and for regenerating bone tissue. Also an object of the invention is a method for obtaining bioactive polymer-ceramic composites with proven pro-regenerative properties for use in bone defect filling and bone tissue regeneration.

Poly(glycerol adipate) (PGA) belongs to the group of biodegradable and bioresorbable aliphatic polyesters, which are obtained by a polycondensation reaction in the presence of the enzyme Candida Antarctica lipase B (CALB) (Jianxu Zhang et al. in Process Biochemistry 49 (2014) 797-806 doi:10.1016/j.procbio.2014.02.006). The use of the enzyme allows the polymerisation reaction to be carried out under relatively mild conditions, the reaction catalyst is non-toxic, the enzyme has high activity, and the enzyme can be recovered (Jun-ichi Kadokawa et al. in Current Opinion in Chemical Biology 14 (2010) 145-153 doi:10.1016/j.cbpa.2009.11.020). The use of enzymes furthermore avoids the use of traditional catalysts containing toxic heavy metals. For this reason, polymers obtained by this technique are often referred to as 'green chemistry polymers'. A method for the synthesis of PGA from divinyl adipate by solvent-assisted enzymatic (CALB) polytransesterification is known from the literature and described in detail. This method yields a so-called PGA prepolymer (pPGA) with a linear chain structure and a weight up to 12kDa (Vincenzo Taresco et al. in Polymer 89 (2016): 41-49 doi:10.1016/j.polymer.2016.02.036, Billie J. Kline et al in Journal of the American Chemical Society 120 (1998) 9475-9480 doi: 10.1021/ja9808907). The structure of the polymer is usually confirmed by FTIR,¹ H NMR and¹³ C NMR, GPC techniques. PGA derivatives formed by enzymatic synthesis of divinyl adipate with xylitol and D-sorbitol are also known from the literature, including poly(xylitol adipate) (PXA) or poly(D-sorbitol adipate) (PDSA) (Muhammad Humayun Bilal et al. in Polymers 8 (2016) 80 doi:10.3390/polym8030080). Other chemically modified PGA derivatives are also known, e.g. with ethylene glycol (Lucila Navarro et al. in Polymer Journal 49 (2017) 625-632 doi:10.1038/pj.2017.30).
PGA and its derivatives are described in the literature for various applications including as drug carriers - in so-called 'drug delivery systems' (Verena M. Weiss et al. in Macromolecular Bioscience 18 (2018) 1700240 doi: 10.1002/mabi.201700240, Paraskevi Kallinteri et al in Biomacromolecules 6 (2005) 1885-1894 doi: 10.1021/bm049200j, Sadie M.E. Swainson et al in Polymers 11 (2019) 1561 doi:10.3390/polym11101561) and formed, for example, into nanometric particles (Sanyogita Puri et al in Journal of Controlled Release 125 (2008) 59-67 doi:10.1016/j.jconrel.2007.09.009). The formation of nanometric spheres for use as drug carriers is a major application of these materials (Verena M. Weiss et al in Macromolecular Bioscience 18 (2018) doi:10.1002/mabi.201700240, Tom Wersig et al in European Journal of Pharmaceutical Sciences 123 (2018) 350-361 doi: 10.1016/j.ejps.2018.07.053, Sadie M.E. Swainson et al in Polymers 11 (2019) 1561 doi:10.3390/polym11101561).

One of the primary functionalities of PGA is the possibility of chemical modification due to the presence of a hydroxyl group in each mer. Chemical functionalisation by reacting the free hydroxyl groups of PGA has been reported in the literature. For this purpose, Steglich esterification was used to conjugate N-acyl-tyrosine with free hydroxyl groups and the substitution of 30% of the functional groups present in the polymer was achieved (Domenico Sagnelli et al. in Coatings 9 (2019) 482 doi:10.3390/coatings9080482). Chemical modifications of the polymer chain with compounds from the diols group (e.g. 1,6-n-hexanediol) and glycerol are also known and described in the literature (Philippa L. Jacob et al. in Polymer 228 (2021) 1239122021 doi:10.1016/j.polymer.2021.123912. Chemical modifications of this polymer with other compounds than those described in the patent application have been reported in the literature. PGA derivatives obtained by chemical modification known from the literature have not been described as components of composites used in bone tissue engineering.

Two-component systems (composites) are known and described in the literature based on bioglass and natural polymers - that is, polymer-ceramic, among which we can mention collagen, gelatine, hyaluronic acid, fibroin, chitosan, cellulose (Rachele Sergi et al. in Materials 13 (2020) 1-38 doi:10.3390/ma13235560). From the group of biodegradable synthetic polymers, bioglass doped poly(glycerol sebacate) composites have so far been described in the literature (Shu Ling Liang et al in Biomaterials 31 (2010) 8516-8529 doi:10.1016/j.biomaterials.2010.07.105).

Three-component systems based on, among others, chitosan, bioglass and a peptide with a sequence (Pro-Glu-Pro-Thr-lle-Asp-Glu-Ser) are also described in the literature. This paper only investigated whether the chitosan/bioglass system could be a carrier for the peptide (Monika Biernat et al. in International Journal of Applied Ceramic Technology 17 (2020) 2807-2816 doi:10.1111/ijac.13609).

Bioglass particles are used in the preparation of bioactive composites with clinical applications in regenerative medicine, tissue engineering and dentistry. On the one hand, their role is to improve the mechanical properties of the material and, on the other hand, they exhibit osteoinductive and osteoconductive properties (when in contact with physiological fluids, hydroxyapatite similar in structure to the natural component of bone is formed on the surface of the bioglass), which has been confirmed by studies and reported in the literature (Helen H. Lu, et al. in Biomaterials 26 (2005) 6323-6334 doi:10.1016/j.biomaterials.2005.04.005, Tadashi Kokubo, et al. in L. Biomed. Mater. Res. 24 (1990) 721-734 doi:10.1002/jbm.820240607, M. Regina Filgueiras et al in J Biomed Mater Res. 27 (1993) 445-453 doi:10.1002/jbm.820270405). The deposition of hydroxyapatite on the surface of biomaterials is usually considered one of the initial steps leading to the formation of a stable bond at the implant/tissue interface. Furthermore, it is believed that the presence of a filler in the form of bioglass particles can positively influence and neutralise acidic degradation products of composite materials (polyesters) (Di Zhang et al. in Materials Chemistry and Physics 111 (2008) 497-502 doi:10.1016/j.matchemphys.2008.04.055).

A number of polymeric composites with applications in tissue engineering and as bone grafting materials have been described in the literature using biodegradable polymers such as poly(lactide-co-glycolide) in Pub: Helen H. Lu et al in Biomaterials 26 (2005) 6323-6334 doi:10.1016/j.biomaterials.2005.04.005, poly(L-lactide) in Pub: Du Juan Zhang et al in Journal of Materials Science: Materials in Medicine 20 (2009) 1971-1978 doi:10.1007/s10856-009-3772-7, poly(e-caprolactone) in publications: Paola Fabbri et al in Composites Science and Technology 70 (2010) 1869-1878 doi:10.1016/j.compscitech.2010.05.029, Patrina S P Poh et al in Biofabrication 5 (2013) 045005 doi:10.1088/1758-5082/5/4/045005, Joaquin Ródenas-Rochina et al Journal of Materials Science: Materials in Medicine 24 (2013) 1293-1308 doi:10.1007/s10856-013-4878-5, poly(glycerol sebacate) as published by Shu-Ling Liang et al in Biomaterials 31 (2010) 8516-8529 doi:10.1016/j.biomaterials.2010.07.105 and bioglass particles as a filler: Mohamed N Rahaman et al in Acta Biomaterialia 7 (2011) 2355-2373 doi:10.1016/j.actbio.2011.03.016, Julian R. Jones in Acta Biomaterialia 9 (2013) 4457-4486 doi:10.1016/j.actbio.2012.08.023, Sekaran Saravanan et al in International Journal of Biological Macromolecules 93 (2016) 1354-1365 doi:10.1016/j.ijbiomac.2016.01.112.

The use of L-lysine diisocyanate ethyl ester in the context of chemical modification to produce biodegradable polyurethanes for tissue engineering applications has been described in the literature. A publication by Cai Wang et al in Polymers 11 (2019) 1927 doi:10.3390/polym11121927 describes the preparation and characterisation of non-toxic and biodegradable polyurethanes based on polyhydroxyalkanoates, polyethylene glycol) and L-lysine diisocyanate.
In contrast, in publications: Jian Ying Zhang et al in Biomaterials 21 (2000) 1247-1258 doi:10.1016/50142-9612(00)00005-3 and Jian-Ying Zhang et al in Tissue Engineering 8 (2002) 771-785 doi:10.1089/10763270260424132 and patent E.J. Beckman et al US 2005/0013793 A1 present the use of L-lysine diisocyanate for the synthesis of polyurethanes. The use of L-lysine diisocyanate in place of commonly used aromatic diisocyanates results in the elimination of undesirable effects due to the release of carcinogenic diamines through degradation, which occurs in the degradation of polyurethanes based on aromatic diisocyanates such as diphenylmethylene diisocyanate or toluene diisocyanate.

The disadvantages of known polymer-ceramic biocomposites for use as bone defect fillers and for bone regeneration are:
- lack of effective pro-regenerative action,
- stiffness and fragility,
- lack of tolerance of the shape fit to the dimensions of the bone defect.

The aim of the invention was therefore to develop such biocomposites for use as filling of bone defects and for bone regeneration so that they have the following properties:
- had proven pro-regenerative bioactive properties,
- were elastomeric materials and thus characterised by elasticity and reversible deformation and the ability to adapt to the dimensions of the bone defect.

These effects were achieved by developing a composition of ternary composites based on poly(glycerol adipate) cross-linked with L-lysine diisocyanate ethyl ester, bioglass particles of one of two types: non-surface-modified bioglass, L-lysine surface-modified bioglass and a peptide with pro-regenerative effects (UG51 peptide). In the bio-composites according to the invention, two types of bioglass particles are used, one selected in the composition: non-surface-modified bioglass or L-lysine surface-modified bioglass.

The bioglass particles selected in the composition are characterised by enhanced bioactivity effect and osteogenic effects.

It was decided to modify and test the effect of bioglass particles covalently functionalised with L-lysine which has been shown to be beneficial in bone regeneration applications. L-lysine promotes osteoblast adhesion and proliferation (Yoshikawa Masataka et al. in Journal of Biomedical Science and Engineering 8 (2015) : 389-398 doi:10.4236/jbise.2015.86037, Liuyun Jang et al. in Journal of Biomaterials Applications 30 (2016): 750-758 doi: 10.1177/0885328215584491) and enhances the osteogenic potential of bone stem cells (Yoshikawa Masataka et al. in Journal of Biomedical Science and Engineering 5 (2012): 587-592, doi:10.4236/jbise.2012.510072).

The essence of the invention is three-component elastomeric composites with pro-regenerative properties based on poly(glycerol adipate) cross-linked with L-lysine diisocyanate ethyl ester, bioglass particles with a chemical composition characterised by enhanced bioactivity and osteogenic effect effect - i.e. one of two types: either bioglass or surface-modified L-lysine bioglass, and a peptide with proven pro-regenerative effects, for use in the filling of bone defects and regeneration of bone tissue. In the description, the abbreviation L-lysine diisocyanate means ethyl ester of L-lysine diisocyanate. The composite has a medical application as a pro-regenerative material for filling bone defects and regenerating bone tissue. As for the amount of individual components in the biocomposite: poly(glycerol adipate) cross-linked with ethyl ester of L-lysine diisocyanate is 77 wt % to 89 wt % relative to the whole, i.e. the ternary composite, the bioglass particles (one type from the selected aforementioned - modified or not) is in an amount of 10-20 wt %, advantageously 10 -12 wt % relative to the whole, and the UG51 peptide is from 1 wt % to 3 wt %, shown on the SEQ1 sequence, relative to the whole. In the example, the amount of 10% wt. bioglass is described.

A composite with pro-regenerative properties for filling bone defects and regenerating bone tissue contains bioglass particles based on SiO₂, CaO and P₂O₅ not surface-modified or bioglass surface-modified with L-lysine. Advantageously, the bioglass contains SiO₂ 60-75 wt %, CaO 10-30 wt % and P₂O₅ 1-8 wt %, most advantageously, the bioglass contains: SiO₂ 70 wt %, CaO 25 wt %, P₂O₅ 5 wt %, relative to the total bioglass, which is 90% below 250 µm in grain size, or covalently modified with L-lysine. Advantageously, the content of bioglass particles of one of the two types: bioglass and L-lysine surface-modified bioglass, is 10% wt. of the total composite.

Advantageously, in composites with a pro-regenerative effect, the molecules of a peptide with a proven pro-regenerative effect are homogeneously distributed throughout the composite material.

Also of essence according to the invention is a method for producing ternary elastomeric biocomposites with pro-regenerative properties based on poly(glycerol adipate) cross-linked with L-lysine diisocyanate ethyl ester, bioglass particles characterised by enhanced bioactivity and osteogenic effect, one type selected from two: bioglass and L-lysine surface-modified bioglass, and an engineered peptide, hereafter referred to as 'UG51' with studied and proved pro-regenerative effects, for use in bone defect filling and bone tissue regeneration.

The ternary composites according to the invention in the form of elastic solid materials with pro-regenerative effects are obtained by thermal cross-linking a mixture of PGA chemically cross-linked with L-lysine diisocyanate ethyl ester, bioglass particles of one of two types: bioglass and L-lysine surface-modified bioglass and UG51 peptide. One step in the development of the biocomposite is the method of introducing the UG51 peptide, with proven pro-regenerative properties, into the composites, leading to the peptide molecules being homogeneously distributed throughout the material, which is advantageous due to the required low release rate of the peptide and ensures a prolonged pro-regenerative effect of the biocomposites.

One step in the formation of the biocomposite, according to the invention, is the preparation of the elastomeric matrix in two stages, by chemical crosslinking of poly(glycerol adipate) with L-lysine diisocyanate ethyl ester in a solvent in the first stage and thermal crosslinking of the reaction product of poly(glycerol adipate) with L-lysine diisocyanate ethyl ester in the second stage. This first stage alone is shown schematically in Formula 1 and further described. Formula 1. Chemical reaction of the cross-linking of poly(glycerol adipate) with the ethyl ester of L-lysine diisocyanate.

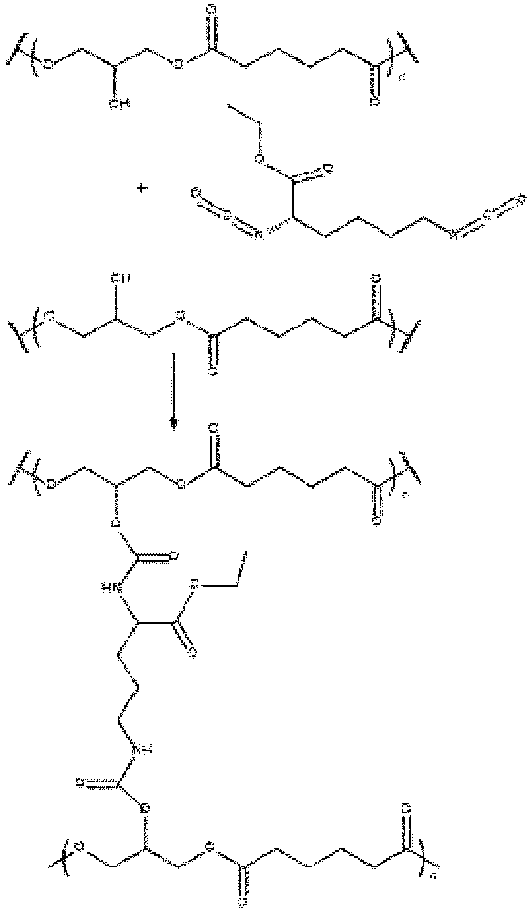

The method of obtaining a composite with pro-regenerative properties for filling bone defects and regenerating bone tissue is based on the fact that chemical crosslinking of poly(glycerol adipate) PGA with ethyl ester of L-lysine diisocyanate (LDI) in tetrahydrofuran is carried out in a first step, with a ratio of isocyanate groups to hydroxyl groups in poly(glycerol adipate) of 0.25 to 1, preferably with an equimolar ratio of isocyanate groups in the ethyl ester of L-lysine diisocyanate to hydroxyl groups in poly(glycerol adipate), at a temperature of 25°C to 60°C for a period of 5 to 24h, preferably at 50°C for 5h. A mixture of the components of the composite is then prepared at room temperature consisting of PGA chemically cross-linked with L-lysine diisocyanate ethyl ester in an amount of 77 wt % to 89 wt %. relative to the total of the ternary composite, bioglass particles unmodified or surface modified with L-lysine in an amount of 10-20 wt %, preferably 10-12 wt % relative to the total of the ternary composite, and a peptide with the SEQ1 sequence having a pro-regenerative effect in an amount of from 1 wt % to 3 wt % relative to the total and the substrates are thoroughly mixed and degassed. The mixture is then thermally cross-linked at 30°C to 50°C, preferably 40°C, until the isocyanate band originating from the ethyl ester of L-lysine diisocyanate disappears.

The entire method - of obtaining the ternary biocomposite according to the invention consists of the methods described below carried out in the order (steps):
1) - Synthesis of a poly(glycerol adipate) prepolymer by the known enzyme-catalysed polytransesterification reaction of divinyl adipate and glycerol.
2) - Synthesis of bioglass by the well-known sol-gel method.
   The method uses a pre-synthesised poly(glycerol adipate) prepolymer and unmodified bioglass.
3) - In the case of the use of covalently modified bioglass, a bioglass particle modification step is performed involving covalent functionalization of the bioglass particle surface with L-lysine, a well-known method for modifying the bioglass particle surface with 3-aminopropyltriethoxysilane APTS and, in a subsequent step, a coupling reaction between the amino groups of APTS and the carboxyl groups of L-lysine using a coupling agent, e.g. 1-ethyl-3-(3-dimethylaminopropyl) carbodiimide hydrochloride (EDC) and N-hydroxysuccinimide (NHS) to increase the yield of the reaction.
4) - Synthesis of UG51 peptide by a known method e.g. conducted on a solid support e.g. SPPS using Fmoc methodology e.g. using an automated microwave synthesiser.
5) - Chemical cross-linking of poly(glycerol adipate) with L-lysine diisocyanate ethyl ester - step described further.
   The fifth step, shown in formula 1, consists in the reaction of the finished prepolymer poly(glyceryl adipate) with the ethyl ester of L-lysine diisocyanate carried out in tetrahydrofuran at an equimolar ratio of isocyanate groups of the ethyl ester of L-lysine diisocyanate and hydroxyl groups in poly(glyceryl adipate), at a temperature of 25°C - 60°C, for a time of 5h - 24h. Advantageously, the chemical cross-linking of PGA with L-lysine diisocyanate ethyl ester is carried out in tetrahydrofuran as solvent, at 50°C for 5h.
6) - Preparation of a ternary mixture consisting of the substrates: PGA chemically cross-linked with L-lysine diisocyanate ethyl ester in an amount ranging from 77 wt.% to 89 wt.% relative to the ternary composite, bioglass particles of one of two types: bioglass and L-lysine-modified bioglass in an amount of 10-20 wt.%, relative to the ternary composite, preferably 10 wt.%-12 wt.%, relative to the ternary composite, and a peptide with pro-regenerative activity in an amount of 1 wt.% to 3 wt.% relative to the ternary composite. The compounding step is carried out by preparing the mixture of biocomposite components at room temperature by thoroughly mixing the components and degassing the mixture in a vacuum dryer before the thermal crosslinking step.
7) - Thermal cross-linking of the ternary mixture carried out at 30°C - 50°C, preferably 40°C until the isocyanate band originating from the ethyl ester of L-lysine diisocyanate disappears. Advantageously, the thermal cross-linking is carried out at 40°C in an air-circulating dryer for 14 days.

In composites with pro-regenerative properties, as an elastomeric matrix, poly(glycerol adipate) chemically cross-linked with L-lysine diisocyanate ethyl ester in a first step according to the method described above - 5) step and, in addition, this chemically cross-linked polymer is then thermally cross-linked in a seventh step in the manufacture of biocomposites, e.g. at 40°C, is used.

In composites with a pro-regenerative effect, the molecules of the peptide with proven pro-regenerative effect are distributed homogeneously throughout the volume of the composite material and to achieve this, the following steps are carried out in the sixth step: the components of the mixture are mixed to obtain a homogeneous distribution of the components in the mixture, and in the seventh step, thermal cross-linking of poly(glycerol adipate) chemically cross-linked with L-lysine diisocyanate ethyl ester is carried out in the presence of bioglass particles and peptide molecules.

The object of the invention is shown in examples 1 to 10, and in the reaction scheme for the chemical cross-linking of poly(glycerol adipate) with the ethyl ester of L-lysine diisocyanate in formula 1. The sequences of the amino acid UG51 are shown in the sequence list.
Figure 1 summarises the cytocompatibility (A, D), proregenerative (B, C) and chemotactic (E) effects of the UG51 peptide demonstrated in the MTT reduction assay, wound healing assay and migration assay, respectively.
Figure 2 summarises the cytocompatibility (A), chemotactic effect (B) and the effect of promoting colonisation by osteoblasts of the PGA_LDI50%_P5_II10%_UG51_1% composite releasing peptide UG51.
Figure 3 depict the release profile of the UG51 peptide from the PGA- LDI50%_P5_II10%_UG51_3% composite.
Figure 4 depict the release profile of the UG51 peptide from the PGA- LDI50%_P5_II- Lys10%_UG51_1% composite.
Figure 5 summarises the cytocompatibility (A), chemotactic effect (B) and the effect of promoting colonisation by osteoblasts (C) of the PGA- LDI50%_P5_II-Lys10%_UG51_1% composite releasing UG51 peptide.
Figure 6 summarises microscopic images 30 days after implantation of the PGA- LDI50%- P5_II-Lys10%_UG51-1% biocomposite: A- transparent implant in the center, limited by bone tissue (40x, stained by V.G.), B- spongy bone tissue surrounding the implant (40x, stained by V.G.), C- on the left edge of the implant, on the right spongy bone tissue (100x, stained by V.G.), D- on the left edge of the implant - phase boundary (400x, stained by V.G.).

The glass transition temperatures of the polymers (T_{g}) were determined from curves from DSC measurements, the thermal stability temperatures corresponding to the 10% mass loss temperature (T_{-10%}) were determined from curves from TGA measurements. DSC and TGA measurements were carried out at a nitrogen flow rate of 60 ml/min. and a heating and cooling rate of 10 °C/min.

### Example 1: PGA synthesis

25.00g of divinyl adipate, 11.61g of glycerol and 1.10g of Novozyme 435 were weighed into a 250ml round-bottomed two-neck flask. 50ml of anhydrous tetrahydrofuran was added and stirred on a magnetic stirrer until the divinyl adipate was dissolved. The freeze-thaw cycle was then repeated three times in liquid nitrogen and vacuum on a Schlenk vacuum-nitrogen line. The mixture was then stirred (250rpm) and heated at 40°C under a reflux condenser on a Schlenka line in a nitrogen atmosphere for 24h. After 24h, the Novozym 435 enzyme was filtered from the reaction mixture on a glass filter. The solvent was evaporated from the filtrate on a rotary evaporator. The product was dried at 95°C for 1h to deactivate any residual enzyme. The product was then dried in a vacuum dryer for 3 days at 40°C. The product was purified by dialysis of a solution of the reaction product in acetone on a MWCO 1kD membrane. Dialysis was carried out for 48h, and the acetone was changed after 24h. Solvent was evaporated from the dialysis solution on a rotary evaporator. The product was dried in a vacuum dryer at 40°C for 24h. The product as an oily colourless liquid with high viscosity was obtained in 75% yield. The structure of the product was confirmed by proton and carbon magnetic resonance (¹H NMR and ¹³C NMR), Fourier transform infrared spectroscopy (FTIR). The thermal properties of the product were characterised by differential scanning calorimetry (DSC) and thermal stability was determined by thermogravimetric analysis (TGA).

**¹H NMR** (acetone-d₆ , 600MHz), δ (ppm): 5.29 (t,1H); 5.09 (m, 1H); 4.89 (1H, m); 4.37 (d, 2H); 4.35 (d, 1H); 4.13 (m, 2H); 4.11 (s, 1H); 4.06 (d, 1H); 3.84 (t, 1H); 3.70 (d, 1H); 3.56 (m, 1H); 2.91 (s, 1H); 2.38 (s, 4H, -CH₂ C=O); 1.65 (s, 4H, -HC -CH₂₂ -).

¹³C NMR (acetone-d₆, 600MHz), δ (ppm): 75.47 (s); 72.20 (s); 69.95 (s); 69.11 (s); 67.22 (s); 65.39 (s); 64.96 (s); 63.65 (s); 63.15 (s); 62.35 (s); 61.98 (s); 60.79 (s); 60.42 (s).

**FTIR-ATR:** The FTIR-ATR spectrum shows bands at 3459cm⁻¹ and 1417cm⁻¹ originating from stretching and bending vibrations of -OH groups. The bands at 2950cm⁻¹, 2874cm⁻¹, 1455cm⁻¹ are characterised by stretching and bending vibrations of C-H bonds located in the PGA main chain. The high-intensity band at 1728cm⁻¹ comes from the stretching vibrations of the carbonyl group (C=O). The bands in the range 1300-1050cm⁻¹ are characterised by stretching and deformation vibrations of the C-O-C bonds.

**DSC and TGA:** T_{g (1st heating)} = -31.6°C; T_{-10%} = 382°C.

### Example 2. synthesis of poly(glycerol adipate) chemically cross-linked with L-lysine diisocyanate ethyl ester (PGA-LDI)

5.00g of PGA was dissolved in 50ml of anhydrous tetrahydrofuran. 2.50ml of L-lysine diisocyanate ethyl ester was dropped into the solution. The reaction solution was stirred on a magnetic stirrer (250rpm) and heated at 50°C under a reflux condenser in a nitrogen atmosphere for 5h. The tetrahydrofuran was then evaporated on a rotary evaporator. The product as an oily colourless liquid was obtained in 100% yield. The structure of the product was confirmed by Fourier transform infrared spectroscopy. The thermal properties of the product were characterised by differential scanning calorimetry and thermal stability was determined by thermogravimetry.

**FTIR-ATR:** The FTIR-ATR spectrum shows bands at 3365cm⁻¹ and 1417cm⁻¹ originating from stretching and bending vibrations of the -OH groups. The bands at 2950cm⁻¹, 2869cm⁻¹ and 1456cm⁻¹ are characterised by stretching and bending vibrations of C-H bonds located in the PGA main chain. The high-intensity band at 1723cm⁻¹ is associated with the presence of a carbonyl group and is shifted towards lower wave numbers relative to the PGA spectrum. At 2252cm⁻¹ a band from stretching vibrations of unreacted isocyanate groups of the ethyl ester of L-lysine diisocyanate is visible. In addition, a new absorption band appears at 1531cm⁻¹ the so-called II amide -CO-NH band, confirming the formation of an amide bond in the reaction of the isocyanate group of the ethyl ester of L-lysine diisocyanate with the hydroxyl group of PGA. In addition, a band is observed at 1021cm⁻¹ from the stretching vibrations of the C-O-C ether bonds originating from the ethyl ester of L-lysine diisocyanate.

**DSC and TGA:** T_{g (1st heating)} = -41.0°C; T_{-10%} = 200°C.

### Example 3. Bioglass P5_II

Bioglass produced by the sol-gel method with a chemical composition of SiO₂ 70 wt.%, CaO 25 wt.%, P₂O₅ 5 wt.% and grain size dv (0.1) 4 µm; dv (0.5) 27 µm; dv (0.9) 77µm. One at a time, 28 ml of anhydrous ethanol was measured into a PTFE beaker and 31.2 ml of C₈H₂₀O₄Si was slowly added using intense stirring with a magnetic stirrer. Then 1.44 ml of C₈H₂₀O₅P₂ was added catalysing the system with 2 M HNO₃. After homogenisation, 28.64 g of a 44.1 wt.% solution Ca(NO₃)₂·4H₂O was added. The whole mixture was homogenised using a magnetic stirrer. The beaker with the reaction mixture was then placed in a drying oven and held at 40°C for 7 days. After each additional 7 days, the drying temperature was increased to 60°C, 80°C, 120°C and 180°C. After holding for 7 days at 180°C, the dried gel was transferred to a ceramic crucible and placed in an electric oven. Heating was carried out at 600°C for 10h and then at 650°C for 15h. After removal from the furnace, the bioglass was crushed in a mechanical mortar to a grain size where 90% of the grains were below 453µm. The crushed glass was then placed in a rotary-vibrating mill with 7mm diameter alundum grinders and ground to a grain size where 90% of the grains were below 77µm.

The grain size analysis of the glass obtained was carried out with a Malvern Instruments Mastersizer 2000 laser analyser, using the low-angle laser light scattering (LALLS) method. The instrument used allows the grain size to be examined over a wide range from 0.1 µm to 2000 µm with an error of 0.5%. The following characteristic values were determined: dv (0.1) - particle size value below which 10% of the population volume of the test sample occurs, dv (0.5) - particle size value below which 50% of the population volume of the test sample occurs, dv (0.9) - particle size value below which 90% of the population volume of the test sample occurs.

LALLS analysis results: dv (0.1) 4 µm; dv (0.5) 27 µm; dv (0.9) 77 µm.

FTIR analysis of the glass produced was performed using a TENSOR27 Bruker FTIR spectrophotometer. In order to prepare a suitable sample for the test (KBr tablet), a weight of approximately 200 mg of dried KBr was mixed with approximately 0.44-0.9 mg of glass sample. Measurements were taken in ATR mode.

**FTIR:** The main absorption bands at 1070 and 798cm⁻¹ correspond to stretching vibrations of [SiO₄]⁴⁻ tetrahedra, the broad absorption band at 1070cm⁻¹ associated with asymmetric Si-O-Si vibrations in tetrahedra and the bands at about 798cm⁻¹ attributed to symmetric Si-O-Si stretching vibrations (conections of [SiO₄]⁴⁻ tetrahedra). Bands at 1220cm⁻¹ corresponding to Si-O-Ca bending vibrations and weak asymmetric double peaks at about 565 and 582 cm⁻¹ corresponding to P-O bending vibrations of PO₄³⁻ groups. Broad bands observed at 460cm⁻¹ from Si-O bending vibrations, weak bands at 1636cm⁻¹ originating from O-H bonds. The broad weakly intense bands visible on the spectrum at 3100-3700cm⁻¹ originate from stretching vibrations of OH bonds of silanol groups (Si-OH) and HO-H bonds of adsorbed water molecules.

### Example 4: Modification of P5_II bioglass with L-lysine (P5_II-Lys)

Bioglass P5_II is modified according to the method described in the patent application entitled "Modified bioglass for the production of bioactive polymer composites and method of modification of bioglass for the production of bioactive polymer composites", P.440261.

A 1.00g of bioglass particles with composition: SiO₂ 70 wt.%, CaO 25 wt.%, P₂O₅ 5 wt.% and grain size dv (0.1) 4µm, dv (0.5) 27µm, dv (0.9) 77µm previously dried at 80°C for 24h and 18ml of 95% aqueous acetone solution is placed in a flask. The whole mixture is stirred with a magnetic stirrer at room temperature for 4h. After this time, the suspension is subjected to centrifugation and the separated bioglass particles are washed 3- times with 95% acetone and then with water and dried at 80°C for 24h. The dried bioglass particles are subjected to a two-step modification.

In the first step, particles washed with acetone and 18ml of 95% ethanol are placed in a round-bottomed flask. The suspension is stirred with a magnetic stirrer for 30min. After this time, 1ml of APTS (0.949g) is added to the suspension and the whole is stirred at room temperature under an inert gas atmosphere using a magnetic stirrer for 4h. After this time, the suspension is subjected to centrifugation to separate the bioglass particles. The particles are washed 3 times with fresh portions of 95% ethanol and dried at 80°C under vacuum for 24h. The presence of APTS on the surface of the bioglass particles was confirmed by FTIR-ATR technique. The FTIR-ATR spectrum of the product shows absorption bands characteristic of the ternary bioglass, the main band at 1027cm⁻¹ from asymmetric stretching vibrations of Si-O-Si bonds of SiO₄ tetrahedra and from antisymmetric stretching vibrations of P-O bonds in PO₄³⁻ groups, a weak band at 1628cm⁻¹ from bending vibrations of HO-H bonds in adsorbed water molecules, a band at 791cm⁻¹ from symmetric stretching vibrations of Si bonds- O-Si bonds connecting SiO₄ tetrahedra, a broad and weakly intense band in the range 3100-3700cm⁻¹ from stretching vibrations of O-H bonds of silanol groups (Si-OH) and HO-H bonds in adsorbed water molecules. The presence of absorption bands at 2850cm⁻¹ and 2925cm⁻¹ corresponding to vibrational vibrations of CH₂ groups and a weak absorption band at 1565cm⁻¹ from bending vibrations of N-H bonds of -NH₂ groups, occurring in the silane precursor molecule APTS, confirms the structure of the product of the first stage of modification of bioglass particles. Quantitative analysis of the APTS content on the surface of the bioglass particles was carried out using the TGA thermogravimetric method. The amount of APTS silane precursor introduced on the surface of the bioglass particles, determined from the difference in total mass loss at 900°C between the unmodified bioglass particles and the product of the first modification stage, is 7.2 wt.%. The modified particles are reacted with L-lysine in the second stage.

For this purpose, 0.0356g of L-lysine; 0.0513g of EDC; 0.0401g of NHS and 10ml of MES buffer (pH=6) are placed in a round-bottom flask and the whole mixture is stirred on a magnetic stirrer 30min to activate the carboxyl groups of L-lysine. After this time, the APTS-modified bioglass particles (0.5g) are dispersed in 10ml of MES buffer (pH=6) and added to the mixture of L-lysine, EDC and NHS. The whole mixture is mixed using a magnetic stirrer for 24h. After this time, the suspension is centrifuged to separate the modified particles, washed 3 times with MES buffer (pH=6) and dried under vacuum at 90°C for 24h. The content of L-lysine on the surface of the APTS- and L-lysine-modified bioglass particles was calculated by thermogravimetric analysis and is 5.5 wt.%. The presence of L-lysine covalently attached by an amide bond is confirmed by the presence on the FTIR-ATR spectrum of a characteristic amide I band from the vibration of the C=O bonds at 1463cm⁻¹.

### Example 5. Synthesis of the pro-regenerative peptide UG51

The peptide was synthesised on a solid support (SPPS) using the Fmoc methodology, using CEM Corporation's Liberty Blue automated microwave synthesiser with a constant flow of reagents on Rink Amide ProTide Resin at a deposition rate of 0.59 mmol/g (1.69 g, 1.0 mmol). A single synthesis cycle using a Liberty Blue microwave synthesiser consisted of the following steps: (1) resin washing and elution of excess reactants using N,N'-dimethylformamide (DMF), (2) deprotection of the Fmoc sheath from the α-amino group of the amino acid using a 20% piperidine solution in DMF (v/v), (3) resin washing and elution of excess reactants using N,N'-dimethylformamide (DMF), (4) coupling of the next amino acid residue using a 3-fold excess of Fmoc-amino acid relative to the resin deposition step using N,N'-diisopropylcarbodiimide (DIC) in the presence of (hydroxyimino)ethyl cyanoacetate (Oxyma Pure). The obtained peptidyl-resin was dried using a vacuum desiccator. The reaction to cleave the peptide from the carrier with simultaneous removal of the side chain sheaths was carried out using a reaction mixture consisting of trifluoroacetic acid (TFA), phenol, water, 1,2-ethanedithiol (EDT) and triisopropylsilane (TIPSI) (91.5:5:5:2.5:1; v/v/v/v/v/v). 10 ml of the mixture was used for 1g of resin. The reaction was carried out for three hours using a laboratory shaker and the resin was then drained over a sieve funnel under reduced pressure. The filtrate was concentrated using a vacuum evaporator. The crude product was precipitated from the mixture using cold diethyl ether. The obtained precipitate was decanted using a glass dipstick and then centrifuged for 20 minutes at 4ºC at 4000 rpm. After decanting in diethyl ether, the precipitate was resuspended in diethyl ether to remove impurities (swirling after each wash), then dried in a vacuum desiccator. The crude and dry product was dissolved in water and subjected to sublimation drying using a lyophiliser. Peptides were subjected to purification using reversed-phase high-performance liquid chromatography (RP-HPLC) using a semipreparative Jupiter^{®} Proteo C12 column (Phenomenex) 4 µm, 90 Å, (21.2 mm x 250 mm). Chromatographic separation was carried out in a linear gradient. The mobile phase was a solvent system:
- A - 0.1M CH₃ COOH; 0.1M CH₃ COONH₄ in HO₂
- B- 65%CH₃ CN in 0.1M CH₃ COOH; 0.1M CH₃ COONH₄ in H₂O (v/v)

The eluent flow rate was 15 ml/min, UV detection at λ = 223 nm.

High-performance reversed-phase liquid chromatography (Nexera X2, Shimadzu) was used to determine the purity of the synthesised peptide using an analytical Kromasil column (4.6 x 250 mm; C-8; 5 µm), volumetric flow rate was 0.5 ml/min, eluents:
- A - 0.1%TFA in H₂O (v/v)
- B- 80% CH₃ CN in 0.1% TFA in H₂O (v/v).

To confirm the mass of the resulting peptide, mass spectra were performed using a Bruker Briflex III MALDI-TOF spectrometer from Bruker Daltonics and/or LCMS-ESI-IT-TOF on a Kromasil C8 column, 5 µm 100 Å (250 mm x 1 mm). The theoretical monoisotopic compound molecular weight, experimental weight and retention time are shown in the table below.

| name | sequence | retention time | theoretical mass | experimental mass |
|---|---|---|---|---|
| UG51 | QAGIVVPLGLYGFGG | 8.66 min | 1446.7131 | 1445.763 |

| | | | | |
|---|---|---|---|---|
| Gln Ala Gly Ile Val Val Val Pro Leu Gly Leu Tyr Gly Phe Gly Gly | | | | |

**TGA:** T_{-10%} = 309.5°C.

### Example 6. Confirmation of the pro-regenerative effect and cytocompatibility of UG51 peptide

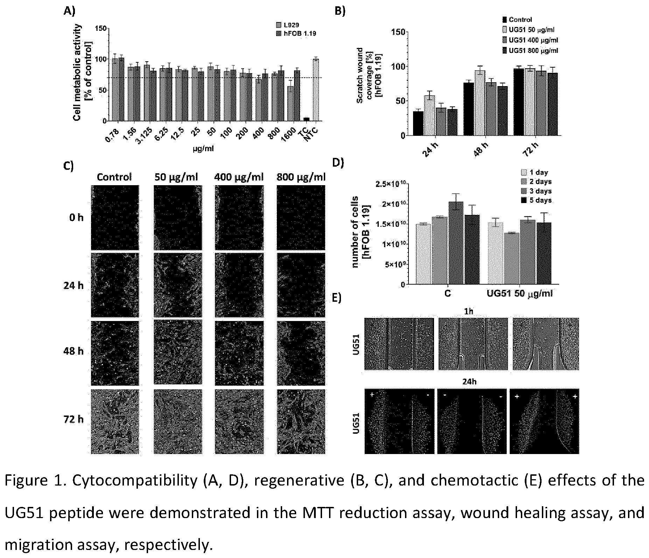

UG51 peptide in the concentration range: 0.8-200 µg/mL has no cytotoxic activity against mouse fibroblasts of the L929 lineage, and within the concentration range of 0.8 to 1000 µg/mL it is cytocompatible with human osteoblasts of the hFoB.1.19 lineage, as supported by cell viability results after 24h exposure to the peptide, in an MTT reduction assay performed according to the relevant standard (ISO 10993:5 2006) (Figure 1A). Furthermore, the UG51 peptide does not interfere with cell viability, which is maintained at physiological levels even after 5 days of incubation (Figure 1D). The results shown in Figure 1B, Figure 1C confirm the pro-regenerative effect of the peptide at the concentration of 50 µg/mL after 48 hours, compared to cultures containing no peptide (Control bar). The monolayer of cells subjected to prior mechanical damage regenerated (healed the wound) significantly faster compared to the physiological rate (Control) as visualised by inverted light microscopy images (Figure 1C). The chemotactic properties of the peptide were also confirmed (Figure 1E).

### Example 7. PGA_LDI50%_P5_II10%_UG51_1%

5,000g of PGA-LDI, 0.056g of UG51 peptide and 0.562g of P5_II bioglass particles with the following composition were weighed into an agate mortar: SiO₂ 70 wt.%, CaO 25 wt.%, P₂O₅ 5 wt.% and grain size dv (0.1) 4µm, dv (0.5) 27µm, dv (0.9) 77µm. The ingredients were mixed in a mortar until a homogeneous dispersion of the particles in the polymer matrix was achieved. The mixture was then degassed in a vacuum dryer and poured into Teflon moulds with wells 6.5mm in diameter and 1.0mm high and cross-linked at 40°C in an air-circulating dryer for 14 days until complete disappearance of the absorption band at 2252cm⁻¹ originating from the isocyanate group. The structure of the product was confirmed by Fourier transform infrared spectroscopy. The thermal properties of the product were characterised by differential scanning calorimetry and thermal stability was determined by thermogravimetry.

**FTIR-ATR:** The FTIR-ATR spectrum shows characteristic intense bands at wave numbers 1724cm⁻¹, 1651cm⁻¹ and 1527cm⁻¹ originating from the carbonyl groups and the amide I, II bands (also indicating the presence of the UG51 peptide). The absence of an absorption band at 2252cm⁻¹ originating from the isocyanate group of the ethyl ester of L-lysine diisocyanate indicates complete rearrangement of the isocyanate groups. The band at 1020cm⁻¹ describes the vibration of the Si-O-Si bonds connecting the SiO₄ tetrahedra and the P-O bonds in the PO₄³⁻ groups occurring in the P5_II bioglass particles, as well as the vibration of the C-O-C bonds occurring in the PGA-LDI structure. The band at 795cm⁻¹ is responsible for the Si-O bond vibrations originating from the P5_II bioglass. Additional absorption bands on the FTIR-ATR spectrum directly indicate the presence of the UG51 peptide. These are bands at wave numbers 1259cm⁻¹ and 2909cm⁻¹ originating from C-H bond vibrations.

**DSC and TGA:** T_{g (1st heating)} = 14.8°C. T_{-10%} = 319°C.

The release of the peptide from the above composite was carried out in water over a time interval of 0 - 4 days. Samples were taken at time points, replacing each with fresh water, time points: 30 min, 1h, 1.5h, 2h, 2.5h, 3h, 4h, 5h, 6h, 24h, 4 days. The experiment was performed in a 24-well plate at 37°C with continuous shaking. For one repetition of the experiment, 4 samples were used (mass of 4 samples = 158.58 mg) - the assumed total amount of peptide that should theoretically be released into solution is approximately 2.1 mg. The amounts of peptide released were beyond the limit of quantification for both UPLC and MALDI MS.

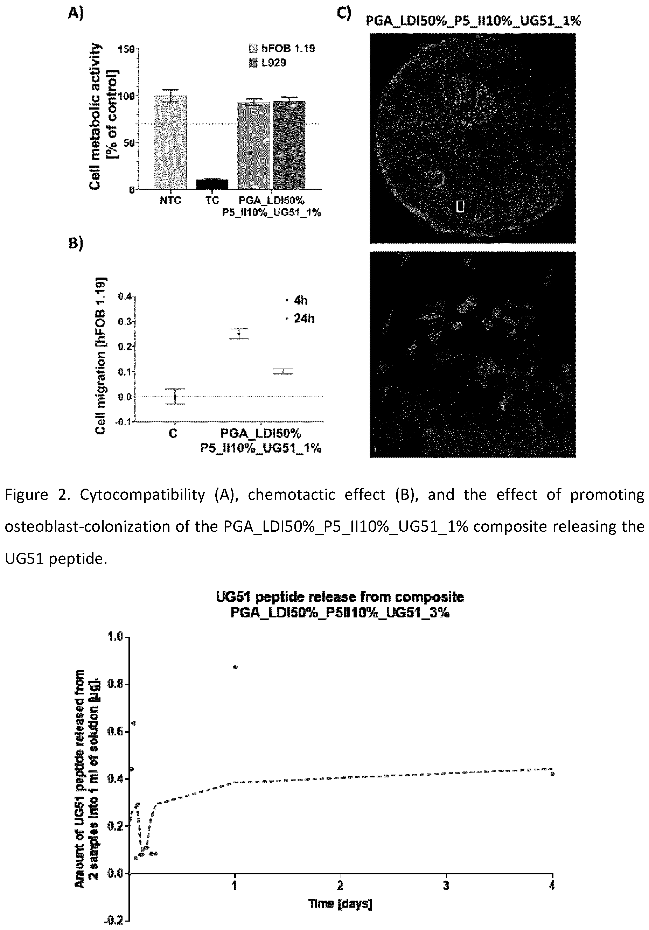

The UG51 peptide-releasing composite is cytocompatible with mouse fibroblast line L929 and human osteoblast line hFoB.1.19, as supported by cell viability results after 24 hours of exposure to the composite, in an MTT reduction assay performed according to the relevant standard (ISO 10993:5 2006) (Figure 2A). In addition, the UG51 peptide-releasing composite acts chemotactically to stimulate osteoblasts to migrate (Figure 2B) and populate the composite (Figure 2C).

### Example 8. PGA_LDI50%_P5_II10%_UG51_3%

The **PGA_LDI50%_P5_II10%_UG51_3%** composite was prepared according to the procedure described in Example 7, except that the preparation used: 5,000g of PGA-LDI; 0.172g of UG51 peptide; and 0.575g of P5_II bioglass particles with the following composition: SiO₂ 70 wt.%, CaO 25 wt.%, P₂O₅ 5 wt.% and grain size dv (0.1) 4µm, dv (0.5) 27µm, dv (0.9) 77µm.

**FTIR-ATR:** The FTIR-ATR spectrum shows characteristic intense bands at wave numbers 1724cm⁻¹, 1651cm⁻¹ and 1527cm⁻¹ originating from the carbonyl groups and the I and II amide bands (also indicating the presence of the UG51 peptide). The absence of an absorption band at 2252cm⁻¹ originating from the isocyanate group of the ethyl ester of L-lysine diisocyanate indicates complete rearrangement of the isocyanate groups. The band at 1016cm⁻¹ describes the vibration of the Si-O-Si bonds connecting the SiO₄ tetrahedra and the P-O bonds in the PO₄³⁻ groups occurring in the P5_II bioglass particles, as well as the vibration of the C O-- C bonds occurring in the PGA-LDI structure. The band at 797cm⁻¹ is responsible for the Si-O bond vibrations originating from the P5_II bioglass. Additional absorption bands on the FTIR-ATR spectrum directly indicate the presence of the UG51 peptide. These are bands at wave numbers 1258cm⁻¹ and 2909cm⁻¹ originating from C-H bond vibrations.

**DSC and TGA:** T_{g (1st heating)} = 11.5°C; T_{-10%} = 317°C.

The release of the peptide from the above composite was carried out according to the procedure described in Example 7, except that 2 samples were used for one repetition of the experiment (mass of 2 samples = 99.14 mg) - the assumed total amount of peptide that should theoretically be released into solution is about 3.15 mg.

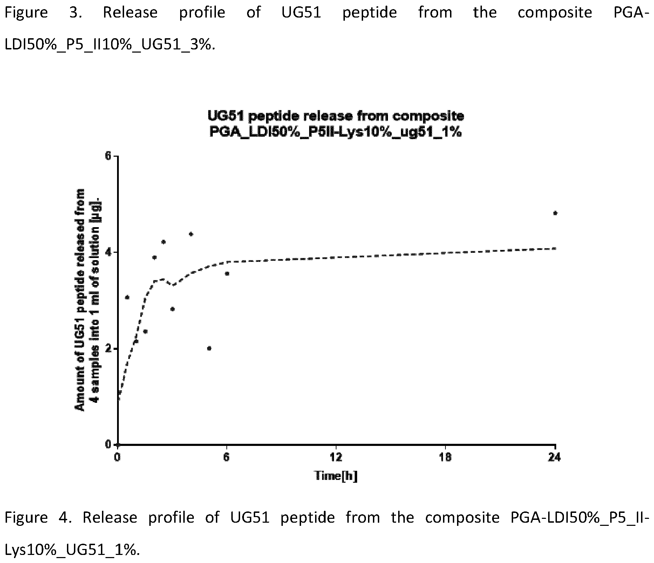

An ejection of peptide was observed immediately after the start of the experiment, after 24h the amount of peptide in the composite had the highest concentration,and remained constant in the following days (Figure 3).

### Example 9. PGA_LDI50%_P5_II-Lys10%_UG51_1%

The **PGA_LDI50%_P5_II-Lys10%_UG51_1%** composite was prepared according to the procedure described in Example 7, except that 5,000g of PGA-LDI; 0.056g of UG51 peptide; and 0.562g of L-lysine-modified P5_II bioglass particles (P5_II-Lys) were used in its preparation.

**FTIR-ATR:** The FTIR-ATR spectrum shows characteristic intense bands at wave numbers 1728cm⁻¹, 1662cm⁻¹ and 1532cm⁻¹ originating from the carbonyl groups and the I and II amide bands (also indicating the presence of the UG51 peptide). The absence of an absorption band at 2252cm⁻¹ originating from the isocyanate group of the ethyl ester of L-lysine diisocyanate indicates complete rearrangement of the isocyanate groups. The band at 1016cm⁻¹ describes the vibration of the Si-O-Si bonds connecting the SiO₄ tetrahedra and the P-O bonds in the PO₄³⁻ groups occurring in the P5_ll-Lys bioglass particles, as well as the vibration of the C-O-C bonds occurring in the PGA-LDI structure. The band at 796cm⁻¹ is responsible for the Si-O bond vibrations originating from the P5_II-Lys bioglass. Additional absorption bands on the FTIR-ATR spectrum directly indicate the presence of the UG51 peptide. These are bands at wave numbers 1259cm⁻¹ and 2907cm⁻¹ originating from C-H bond vibrations.

**DSC and TGA:** T_{g (1st heating)} = 15.4°C; T_{-10%} = 313°C.

The release of the peptide from the above composite was carried out according to the procedure described in Example 7, except that 4 samples were used for one repetition of the experiment (mass of 4 samples = 158.58 mg) - the assumed total amount of peptide that should theoretically be released into solution is about 2.1 mg.

Peptide ejection was observed for about 6h, after which the amount of peptide remained constant (Figure 4).

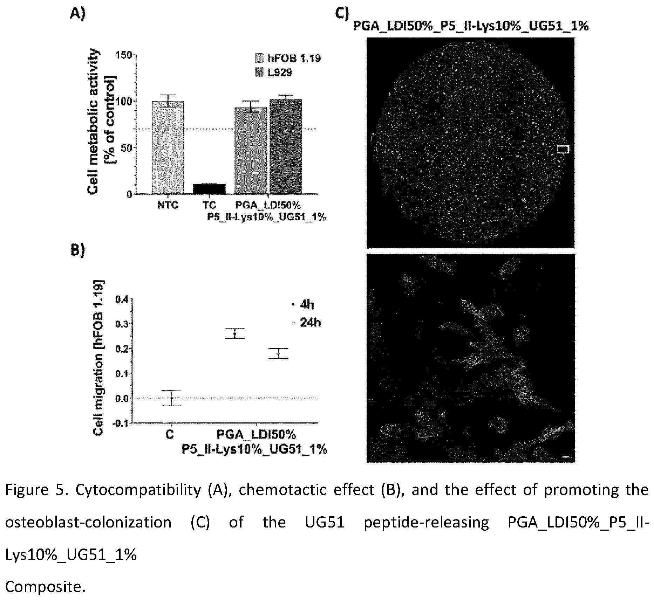

The lysine-modified UG51 peptide-releasing composite is cytocompatible with mouse fibroblast line L929 and human osteoblast line hFoB.1.19, as supported by cell viability results after 24 hours of exposure to the composite, in an MTT reduction assay performed according to the relevant standard (ISO 10993:5 2006) (Figure 5A). In addition, the UG51 peptide-releasing composite acts chemotactically to stimulate osteoblasts to migrate (Figure 5B) and populate the composite (Figure 5C).

### Example 10. PGA_LDI50%_P5_II-Lys10%_UG51_3%

The **PGA_LDI50%_P5_II-Lys10%_UG51_3%** composite was prepared according to the procedure described in Example 7, except that 5,000g of PGA-LDI; 0.172g of UG51 peptide and 0.575g of L-lysine-modified P5_II bioglass particles were used in its preparation.

**FTIR-ATR:** The FTIR-ATR spectrum shows characteristic intense bands at wave numbers 1728cm⁻¹, 1652cm⁻¹ and 1530cm⁻¹ originating from the carbonyl groups and the I and II amide bands (also indicating the presence of the UG51 peptide). The absence of an absorption band at 2252cm⁻¹ originating from the isocyanate group of the ethyl ester of L-lysine diisocyanate indicates complete rearrangement of the isocyanate groups. The band at 1021cm⁻¹ describes the vibration of the Si-O-Si bonds connecting the SiO₄ tetrahedra and the P-O bonds in the PO₄³⁻ groups occurring in the P5_II-Lys bioglass particles, as well as the vibration of the C-O-C bonds occurring in the PGA-LDI structure. The band at 799cm⁻¹ is responsible for the Si-O bond vibrations originating from the P5_II-Lys bioglass. Additional absorption bands on the FTIR-ATR spectrum directly indicate the presence of the UG51 peptide. These are bands at wave numbers 1259cm⁻¹ and 2925cm⁻¹ originating from C-H bond vibrations.

**DSC and TGA:** T_{g (1st heating)} = 13.7°C. T_{-10%} = 322°C.

### Example 11. Acute toxicity testing of the biocomposite PGA-LDI50%-P5_II-Lys10%_UGS1-1%.

Acute toxicity testing of the PGA-LDI50%-P5_II-Lys10%_UG51-1% biocomposite was performed according to the recommendations of PN-EN 10993-11: 2009 'Biological evaluation of medical devices - Part 11: Tests for systemic toxicity'. Samples of the PGA-LDI50%-P5_II-Lys10%_UG51-1% composite were formed into 10mm x 0.5mm discs. A systemic acute toxicity study was conducted on 10 albino Swiss strain, male mice, allocating 5 animals to the test biocomposite and 5pc to the control group. The initial mean body weight of the animals was 35.68 (±1.80) in the control group and 37.88 (±1.90) in the B3 group, respectively. The animals, housed in groups, were subjected to handling training during the acclimatisation period in order to become accustomed to the touch of the experimenter. The experiments were carried out after intraperitoneal injection of aqueous extracts from the test biocomposite at a volume of 50ml/kg body weight of mice (i.e. an average of approximately 1.75cm³ in individual mice) in the test group and similarly -injection with saline for injection in the control group. The extract from the test biocomposite was prepared after flooding the samples - with saline solution for injection at a ratio of the bilateral surface area of the samples to the volume of the extraction mixture - 3cm²/ml. The extracts were then incubated with a parallel control, which was saline - solution for injection at 37°C for 72h. Observations of the animals after injection were carried out for 7 days and included changes in appearance, behavioural abnormalities, stunted weight gain and mortality. Mice were weighed daily, normal function was observed, feed and water consumption were measured, and differences in weight gain between animals of the test and control groups were assessed. No physical or behavioural changes indicative of disease were found in either the test or control groups during the health observations. Each physical examination considered the test animal to be clinically healthy. During the 7-day experimental (post-inoculation) period, there were no behavioural deviations or any signs of disease in the animals, either in the control group or the test group. All mice were considered clinically healthy. Feed and water consumption and body weight gain in the test groups were comparable to the data obtained in the control group, and the differences were not statistically significant. All mice survived until the scheduled post-mortem date. There were no abnormalities in behaviour, appearance or differences in weight gain between the test groups and the control group. After 7 days, euthanasia (with pentobarbital) and postmortem examinations were performed. Post-mortem examinations of the animals, both from the control group and the test group, revealed no skin lesions, skeletal or muscular changes. The natural body orifices (external ear, eyes, nostrils, mouth, anus) and external genitalia showed no lesions. In the peritoneal cavity and thorax, all organs were positioned correctly, with preserved anatomical shape, colour and size. None of the visually inspected organs showed macroscopic pathological changes. No pathological changes were found at autopsy. On the basis of tests carried out on the basis of EN ISO 10993- 11: Biological evaluation of medical devices- Part 11: Tests for systemic toxicity, it was concluded that the samples of the tested biocomposite do not show systemic acute toxicity.

### Example 12. Studies of local bone response after implantation of PGA-LDI50%-P5_II-Lys10%_UG51-1% biocomposite

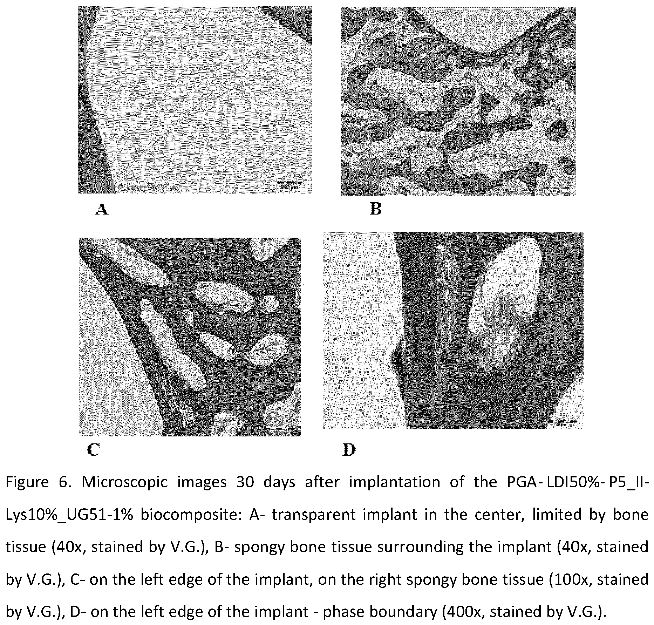

Samples of the PGA-LDI50%-P5_II-Lys10%_UG51-1% biocomposite for testing were cylindrical in shape, measuring 2mm x 6mm, and were radiation sterilised. For the PGA-LDI50%-P5_II-Lys10%_UG51-1% composite, biocompatibility was tested at the in vivo level using a rabbit model (New Zealand rabbits, both sexes, 3 animals per group). The studies were performed in accordance with PN-EN-ISO 10993-6: "Biological evaluation of medical devices" part 6: "Tests for local reaction after implantation" and PN-EN-ISO 10993-11: "Biological evaluation of medical devices" part 11: "Tests for systemic toxicity" (consent of the bioethics committee for the experiments number 83/2019/P1). The study plan included: implantation into the femur of rabbits (2 implants each were implanted into the ileum), macroscopic (postmortem) and histological examinations at 30, 60 and 90 days after surgery, and radiological examinations. After implantation of the composite under study, observation of the rabbits' general health was carried out, with particular emphasis on post-operative wound healing, active and passive mobility of the hip joint and feed intake. During the dissections performed, the post-operative wound and the appearance of the tissues at the site of implantation of the specimens were evaluated macroscopically first, followed by an assessment of the appearance of selected internal organs. Subsequently, femurs with implants were collected for further histological and radiological examination. After implantation of the PGA-LDI50%-P5_II-Lys10%_UG51-1% composite at observation times ranging from 30 to 90 days, the condition of the animals did not deviate from normal. The animals retained active and passive mobility in the hip joints. Post-operative wounds healed by rhytidrosis. On postoperative examination after implantation of the PGA-LDI50%-P5_II-Lys10%_UG51-1% biocomposite, bulging was found in isolated cases, no fluid accumulation was observed, and the wounds healed normally. On microscopic examination, the composite was visible up to 90 days after implantation. On histological examination 30 days after implantation of the PGA-LDI50%-PS_II-Lys10%_UG51-1% biocomposite, the implant was quite sharply demarcated from the surrounding tissues and surrounded by a band of bone tissue. In the surrounding spongy bone tissue, osteoblast activity was evident. Up to 70% of the surrounding bone was composed of bone beads (Figure 6). 60 days and 90 days after biocomposite implantation, the microscopic image was similar to that after 30 days. There were no signs of biodegradation around the biocomposite until day 90.

### UG51 peptide sequence

<110> University of Gdansk, Institute of Biotechnology and Molecular Medicine, Lukasiewicz Research Network, Wroclaw University of Science and Technology, University of Lodz
<120> Polymer-ceramic biocomposites with pro-regenerative properties for filling bone defects and regenerating bone tissue and method of obtaining them
<160> 1
<210> 1
<211> 15
<212> ug51
<213> Artificial sequence
<220>
<223> A peptide designed on the basis of a human cystatin C protein sequence and a sequence specific for metalloproteinase and osetogenic growth factor.
<400>
1 15
Gln Ala Gly Ile Val Val Val Pro Leu Gly Leu Tyr Gly Phe Gly Gly

## Claims

1. Composite with pro-regenerative properties for filling bone defects and regenerating bone tissue based on bioglass and natural polymers, **characterised in that** the composite is based on poly(glycerol adipate) cross-linked with L-lysine diisocyanate ethyl ester, and further comprising particles of SiO₂, CaO and P₂O₅ based non-surface-modified bioglass or L-lysine surface-modified bioglass and a peptide with the sequence shown in SEQ 1 with a pro-regenerative effect, the poly(glycerol adipate) cross-linked with L-lysine diisocyanate ethyl ester being from 77 wt.% to 89 wt.% of the total ternary composite, the bioglass particles are in an amount of from 10 wt.% to 20 wt.%, preferably from 10 wt.% to 12 wt.% relative to the total ternary composite, while the peptide is from 1 wt. % to 3 wt. % relative to the total ternary composite.

2. Composite according to claim 1, wherein the bioglass contains SiO₂ in an amount of 60-75% by weight, CaO in an amount of 10-30% by weight and P₂O₅ in an amount of 1-8% by weight.

3. Composite according to claim 2, wherein the bioglass contains: SiO₂ 70% by weight, CaO 25% by weight, P₂O₅ 5% by weight, relative to the ceramic part of the bioglass, the grain size of which is 90% below 250 µm, or covalently modified with L-lysine.

4. Composite according to claim 1, wherein the content of bioglass particles of one type is 10% by weight in relation to the total composite.

5. Composite according to claim 1, wherein the composite the peptide is homogeneously distributed throughout the volume of the composite material.

6. Method of preparing composite with pro-regenerative properties for filling bone defects and regenerating bone tissue based on bioglass and natural polymers, **characterised by** the fact that the chemical crosslinking of poly(glyceryl adipate) PGA with ethyl ester of L-lysine diisocyanate (LDI) in tetrahydrofuran is carried out in the first step, with a ratio of isocyanate groups to hydroxyl groups in poly(glycerol adipate) of 0.25 to 1, preferably with an equimolar ratio of isocyanate groups in the ethyl ester of L-lysine diisocyanate to hydroxyl groups in poly(glycerol adipate) at a temperature from 25°C to 60°C for a period of time from 5 to 24 hours, preferably at 50°C for 5 hours., and then a mixture of the components of the composite is prepared at room temperature consisting of PGA chemically cross-linked with L-lysine diisocyanate ethyl ester in an amount of 77 wt.% to 89 wt.%. relative to the total of the ternary composite, particles of non-surface-modified or L-lysine-modified bioglass in an amount of 10-20 wt.% relative to the total of the ternary composite, advantageously 10-12 wt.% relative to the total of the ternary composite, and a peptide of SEQ1 sequence with a pro-regenerative effect in an amount of 1 wt.%. to 3 wt.% relative to the total weight of composite. The substrates are thoroughly mixed and degassed, followed by thermal cross-linking of the mixture carried out at 30°C to 50°C, preferably at 40°C, until the isocyanate band originating from the ethyl ester of L-lysine diisocyanate disappears.

7. Method according to claim. 5, wherein the chemical cross-linking of PGA with the ethyl ester of L-lysine diisocyanate is carried out in tetrahydrofuran as solvent, at 50°C for 5h.

8. Method according to claim. 5, wherein the thermal crosslinking of the mixture is carried out until the isocyanate band at 2252cm⁻¹ originating from the ethyl ester of L-lysine diisocyanate disappears.

9. Method according to claims. 5 or 8, wherein the thermal cross-linking of the mixture is carried out for 3 days to 14 days.

10. Method according to claim. 9, wherein the thermal cross-linking is carried out for 14 days.
